# DEMANDE DE BREVET EUROPEEN

(11) **EP 2 774 650 A1**
(43) Date de publication de la demande: **10.09.2014**
(21) Numéro de dépôt: 13157635.7
(22) Date de dépôt: 04.03.2013
(51) Int. Cl.: A61M 39/16, A61M 39/18, A61M 39/20

(54) **Ensemble connecteur capuchon**

(71) Demandeur: Debiotech S.A., 1004 Lausanne (CH)
(72) Inventeur: Neftel, Frédéric, 1005 Lausanne (CH)
(74) Mandataire: Grosfillier, Philippe

(57) **Abrégé**

Ensemble connecteur / capuchon comprenant des moyens de verrouillage et des moyens de couplage permettant de décapuchonner le connecteur avec une seule main et de préférence sans contact du capuchon avec la main du praticien.

## Description

### Domaine de l'invention

L'invention concerne un ensemble connecteur capuchon pouvant être utilisé dans le domaine médical ainsi qu'une méthode de décapuchonnage. Ladite invention permet d'éviter certaine manipulation qui risquerait de contaminer tout ou partie de la ligne fluidique comprenant ledit connecteur.

### Etat de la technique

Dans le domaine médical mais pas uniquement, il est parfois utilisé des connecteurs stériles afin de relier deux lignes fluidiques qui doivent restées aseptiques. Ces connecteurs peuvent être munis de capuchons permettant de fermer les parties à protéger du connecteur afin d'éviter toute contamination avant la connexion desdits connecteurs.

De façon générale lors de la connexion des deux connecteurs, la stérilité doit être assurée sur au moins les parties protégées par le capuchon et jusqu'à au moins la connexion complète des connecteurs. Dit autrement, lorsque que les connecteurs sont décapuchonnés mais pas encore connectés, le praticien ou le patient doit manipuler avec une grande précaution afin de garantir qu'il n'a pas contaminé, de façon non intentionnelle, les connecteurs.

En outre, la connexion de deux lignes fluidiques peut nécessiter l'utilisation de deux connecteurs distincts, chacun étant monté sur une ligne fluidique. Ainsi pour les relier, il faut dans un premier temps décapuchonner les connecteurs puis, dans un deuxième temps, connecter lesdits connecteurs ensembles. Toutefois, afin de ne pas contaminer les connecteurs, une fois décapuchonnés les connecteurs ne doivent être reposés pendant le décapuchonnage de l'autre. Les deux connecteurs doivent ainsi être manipulés ensembles et il est indispensable de toujours maintenir un connecteur pendant le décapuchonnage de l'autre connecteur.

A titre d'exemple, des lignes fuidiques doivent être reliées entre elles par tout patient atteint d'insuffisance rénale et nécessitant une dialyse péritonéale à domicile. Afin d'éviter les risques de contamination, responsables de péritonites infectieuses, certains appareils (SleepSafe de Fresenius) préconisent la connection automatisée. Toutefois le tiroir ou l'on place les connecteurs afin d'assurer leur connexion automatisée peuvent être souillés, réalisant alors un risque encore plus grand de contamination. D'autres dispositifs sont équipés, sur la face externe du connecteur, d'une bague (système Baxter) permettant d'utiliser l'un des doigts de l'autre main (auriculaire) pour retirer le capuchon tandis que chacune des mains tient une ligne à connecter. Un tel système ne garantit pas l'absence de comtamination, étant donné la proximité des doigts avec la face externe du connecteur et rends les manipulations difficiles à expliquer au patient.

Certains brevets revendiquent des ensembles connecteur/capuchon qui permettent de maintenir les connecteurs tout en les décapuchonnant. Par exemple, la demande PCT publié sous le numéro WO 83/00622 ainsi que le brevet européen EP 0 621 053 divulguent des capuchons fixés aux connecteurs par l'intermédiaire d'un moyen formant une charnière. Ces connecteurs peuvent être décapuchonnés simplement avec le pouce et être refermé. Cependant, le connecteur ne comprenant pas toujours de moyens de protection contre le contact du pouce avec la partie initialement protégée par le capuchon, lors de la manipulation, le pouce peut contaminer le connecteur. En effet, en poussant le capuchon, le pouce peut entrer en contact avec la partie initialement protégée ou proche de ladite partie et donc la contaminer. De plus, un tel système ne permet pas de savoir si le connecteur a déjà été utilisé ou simplement décapuchonné temporairement. Ainsi, le connecteur peut avoir été contaminé sans que le praticien ne le sache. Ce type de dispositif ne peut donc assurer que les connecteurs ne sont pas contaminés. En résumé, ce connecteur peut donc être maintenu et décapuchonné avec une seule main, toutefois les risques de contamination par le praticien ou le patient sont importants.

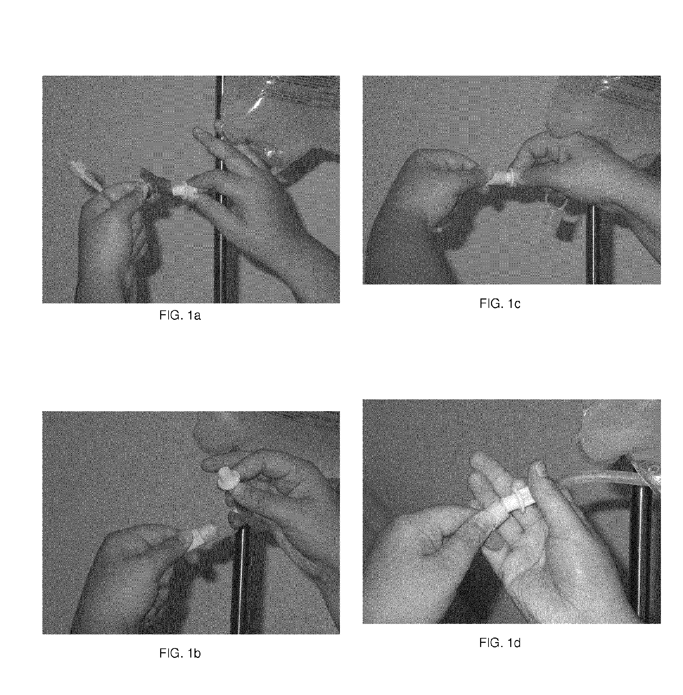

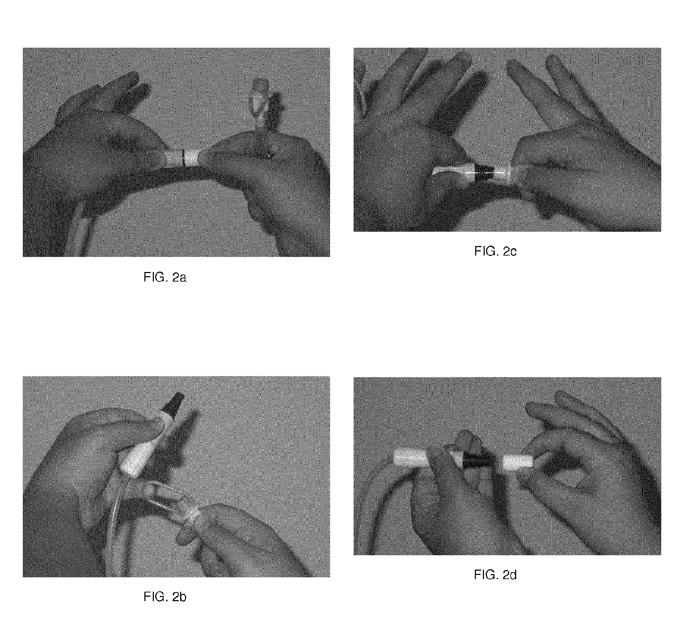

La demande PCT publiée sous le numéros WO 94/28855 et le brevet US 4,573,980 divulguent un capuchon permettant d'éviter le contact de tout élément avec la partie initialement protégée du connecteur. Ainsi, le capuchon comprend un anneau dans lequel un doigt peut y être glissé. Grâce à cet anneau, le connecteur peut être décapuchonné en tirant sur cet anneau. Ainsi, le praticien ne touche pas la partie protégée du connecteur. De façon plus détaillé, les figures 1a à 1c et 2a à 2c décrivent la manipulation nécessaire pour décapuchonner et connecter les connecteurs.

La figure 1a expose deux connecteurs, le premier est maintenu dans la main gauche du praticien et le deuxième dans sa main droite. Le deuxième connecteur est décapuchonné en maintenant ledit connecteur avec le pouce et l'index tandis que les autres doigts de la main droite restent éloignés dudit connecteur, ces doigts forment alors « une aile de papillon ». La main gauche maintien le premier connecteur tout en retirant le capuchon du deuxième connecteur. Dans la figure 1b, le premier connecteur est décapuchonné (grâce à l'anneau) par la main droite qui maintient en même temps le deuxième connecteur déjà décapuchonné. Dans la figure 1c, les deux connecteurs sont connectés en gardant les mains et doigts le plus éloigné possible de l'extrémité distale des connecteurs. La figure 1d expose une façon de connecter à proscrire car les doigts risquent de contaminer les connecteurs.

Les figures 2a à 2d exposent un autre ensemble connecteur/capuchon. Dans la figure 2a, lors du décapuchonnage du premier connecteur, la main gauche maintient le premier connecteur en formant une aile de papillon pendant que la main droite dévisse le capuchon du premier connecteur tout en maintenant éloigné le deuxième connecteur muni de son capuchon. Dans la figure 2b, le premier connecteur est décapuchonné et maintenu de façon à ne pas contaminer la partie initialement protégé par le capuchon tout en arrachant le capuchon du deuxième connecteur grâce à l'anneau de ce dernier avec un doigt de la main gauche, de préférence l'auriculaire. Dans la figure 2c, les deux connecteurs sont connectés en gardant les doigts des deux mains formant des ailes de papillon afin d'assurer de ne pas contaminer les connecteurs. Il est noté en figure 2d une manipulation à proscrire où les doigts de la main gauche peuvent contaminer la partie initialement protégée du premier connecteur lorsque celui-ci est décapuchonné.

En résumé, la demande PCT publié sous le numéros WO 94/28855 et le brevet US 4,573,980 divulguent un type de connecteur qui doit être maintenu par une main et décapuchonné par une autre, tout en faisant attention au placement de ces doigts. Ainsi, durant la manipulation, les risques de contaminations sont nombreux et la méthode de connexion contraignante voir laborieuse. Les praticiens doivent impérativement être formés pour ces manipulations qui ne sont pas évidentes.

Fort de ce constat, de nouveau dispositif ont été créé en partant du postulat que la manipulation par le praticien est un facteur de risque important. Ainsi la demande PCT publiée sous le numéro WO 2009/006507 divulgue un dispositif automatique qui permet de décapuchonner les connecteurs et de connecter les lignes fluidiques sans l'aide du praticien. Toutefois, il s'est avéré que ce genre de dispositif n'assurait pas la stérilité des connecteurs. En effet, les éléments qui protègent le système de connexion ne peuvent pas être aseptisé après chaque utilisation et donc ces éléments de protection peuvent engendrer une propagation de la contamination bien plus importante que le système manuel.

La manipulation par le praticien ou le patient reste ainsi le meilleur moyen de garantir la non contamination de l'ensemble. Toutefois, la manipulation des dispositifs existants restent laborieuses et non évidentes engendrant de nombreux risque de contamination.

### Description générale de l'invention

La présente invention a pour objet de réaliser un connecteur stérile capuchonné dont la manipulation est simplifiée et permet d'éliminer les inconvénients des connecteurs connus cités plus haut.

La présente invention est décrite et caractérisé par la ou les revendications indépendantes, tandis que les revendications dépendantes décrivent d'autres caractéristiques de l'invention.

Le présent document décrit un ensemble connecteur/capuchon pouvant être utilisé dans le domaine médical. Ledit connecteur comprend un corps comportant une extrémité proximale connectée à une ligne fluidique et une extrémité distale façonnée de manière à permettre une connexion avec un autre connecteur ou un autre système / dispositif. Le corps est en outre composé d'un passage d'écoulement de fluide permettant une connexion fluidique entre l'extrémité distale et ladite ligne fluidique. Le capuchon protège au moins ledit passage de manière à protéger ledit passage.

Ledit capuchon et/ou ledit connecteur comprennent des moyens de verrouillage permettant de maintenir au moins temporairement et solidement ledit capuchon audit connecteur.

L'ensemble comprend des moyens permettant de décapuchonner le connecteur de façon simple et évitant toute contamination de la partie à protéger. Ces moyens peuvent être des moyens d'éjections, des moyens de libération, des moyens de couplage et/ou tous autres moyens permettant de décapuchonner facilement le connecteur. Lesdits moyens peuvent permette par exemple de décapuchonner ledit connecteur avec une seule main ou de décapuchonner un connecteur avec l'aide d'un autre ensemble connecteur/capuchon.

### Liste des figures

Pour permettre une meilleure compréhension de l'invention, il sera décrit un ou plusieurs modes de réalisation illustrées par les figures jointes à ce document. Il va de soi que l'invention ne se limite pas à ces modes de réalisation.
**Figures 1a à 1d** **et** **2a** **à** **2d** exposent l'utilisation de connecteurs de l'état de l'art.
**Figure 3** schématise un ensemble connecteur capuchon
**Figure 4a** schématise un système de connecteur
**Figures 4b à 4d** schématisent les actions nécessaire pour le décapuchonnage
**Figure 5** expose différents codages des moyens de couplage
**Figure 6** schématise un autre mode de réalisation d'un ensemble connecteur capuchon
**Figure 7** illustre un autre mode de réalisation d'un système de connecteur
**Figures 8a à 8c** exposent une différente vue du connecteur femelle et de son capuchon
**Figures 9a à 9c** exposent une différente vue du connecteur male et de son capuchon
**Figure 10** exposent un système de connecteur couplé par les capuchons
**Figure 11** schématise les actions nécessaire pour le décapuchonnage et la connexion des connecteurs

### Liste des références numériques utilisées dans les figures

| | |
|---|---|
| 1 | Ensemble connecteur capuchon |
| 2 | Ligne fluidique |
| 3 | Connecteur |
| 4 | Capuchon |
| 5 | Extrémité distale du connecteur |
| 6 | Extrémité proximale du connecteur |
| 7 | Extrémité distale du capuchon |
| 8 | Extrémité proximale du capuchon |
| 9 | Corps du connecteur |
| 10 | Corps du capuchon |
| 11 | Moyens de couplage. |
| 12 | Système de connecteur |
| 13 | Moyens de verrouillage |
| 14 | Moyens de retenu |
| 15 | Moyens de libération |
| 16 | Déclencheur |
| 101 | Ensemble capuchon / connecteur femelle (premier ensemble) |
| 102 | Ensemble capuchon / connecteur male (deuxième ensemble) |
| 103 | Connecteur femelle (premier connecteur) |
| 104 | Connecteur male (deuxième connecteur) |
| 105 | Capuchon du connecteur femelle (premier capuchon) |
| 106 | Capuchon du connecteur male (deuxième capuchon) |
| 107 | Corps du connecteur |
| 108 | Membrane |
| 109 | Filetage |
| 110 | Passage |
| 111 | Extrémité proximale |
| 112 | Extrémité distale |
| 113 | Aillette |
| 114 | Ergot |
| 115 | Elément cylindrique |
| 116 | Moyens de couplage |
| 117 | Fente |
| 118 | Lame élastique |
| 119 | Butée |
| 120 | Elément faisant saillit sur la lame élastique |
| 121 | Extrémité distale du capuchon |
| 122 | Extrémité proximale du capuchon |
| 123 | Axe principale |
| 124 | Butée |

### Description détaillée de l'invention

Dans le présent document, la description détaillée de l'invention comporte des modes de réalisation de dispositifs, de systèmes et de méthodes présentés à titre d'illustration. Il est bien entendu que d'autres modes de réalisation sont envisageables et peuvent être apportées sans s'écarter de la portée ou l'esprit de l'invention. La description détaillée qui suit, par conséquent, ne doit pas être prise dans un sens limitatif.

Sauf indication contraires, les termes scientifiques et techniques utilisé dans le présent document ont des significations couramment utilisés par l'homme du métier. Les définitions apportées dans ce document sont mentionnées afin de faciliter la compréhension des termes fréquemment utilisés et ne sont pas destinées à limiter la portée de l'invention.

Les indications de direction utilisées dans la description et les revendications, telles que "haut", "bas", "gauche", "droite", "supérieur", "inférieur", et autres directions ou orientations sont mentionnées afin d'apporter plus de clarté en référence des figures. Ces indications ne sont pas destinées à limiter la portée de l'invention.

Dans le présent document, le terme « extrémité distale » doit être compris comme l'extrémité la plus éloigné d'une référence. En particulier, si la référence est la ligne fluidique du connecteur, l'extrémité distale d'un connecteur est l'extrémité qui est la plus éloigné de sa ligne fluidique. Cette extrémité peut être également qualifié d'extrémité « libre » car elle peut être contrairement à l'extrémité proximale, elle n'est pas directement relié à la ligne fluidique. En outre, l'extrémité distale d'un capuchon est l'extrémité qui est la plus éloigné de la ligne fluidique.

Inversement, le terme « extrémité proximale » doit être compris comme l'extrémité la plus proche d'une référence. Ainsi, l'extrémité proximale d'un connecteur est l'extrémité qui est directement reliée à sa ligne fluidique et l'extrémité proximale d'un capuchon est l'extrémité qui est le plus proche de la ligne fluidique du connecteur.

Le terme praticien doit être compris au sens large comme celui qui est formé à manipuler les connecteurs et comprends aussi bein une membre du corps médical qu'un patient éventuel.

Ainsi, l'extrémité distale du connecteur peut être en contact avec l'extrémité proximal du capuchon alors que l'extrémité distale du capuchon et l'extrémité proximale du connecteur représente les deux extrémités de l'ensemble connecteur/capuchon. Préférentiellement, les extrémités distales des connecteurs sont façonnées de manière à connecter deux connecteurs.

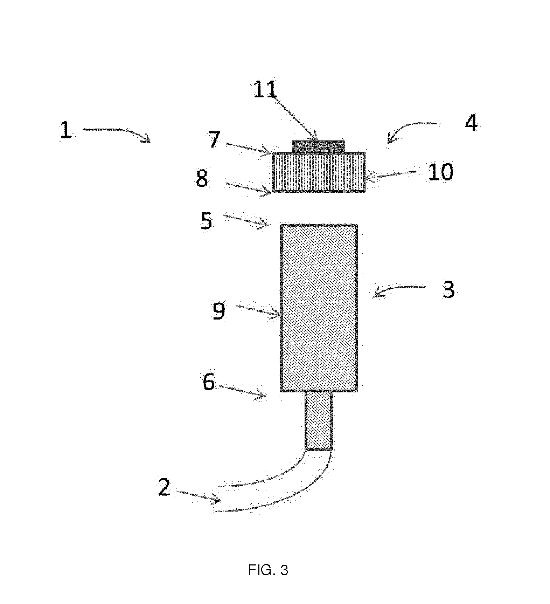

Dans un mode de réalisation exposé en figure 3, l'ensemble connecteur/capuchon (1) comprend un connecteur (3) et un capuchon (4). Ledit connecteur (3) comporte un corps (9) définissant un passage d'écoulement de fluide protégé par ledit capuchon (4) disposé à l'extrémité distale (5) dudit connecteur (3). L'ensemble (1) comprend des moyens de verrouillage (non exposés sur la figure 1) conçus pour maintenir au moins temporairement et solidement le capuchon (4) au connecteur (3). Le capuchon (4) comprend en outre des moyens de couplage agencés de manière à coupler ledit capuchon avec un élément comportant également des moyens de couplage. Préférentiellement, lesdits moyens de couplage (11) permettent un couplage au moins pendant que le praticien désactive les moyens de verrouillage afin de décapuchonner ledit connecteur (3) sans avoir à « toucher » le capuchon (4).

Dans un mode de réalisation, le capuchon (4) se couple à un autre dispositif ou système. Par exemple, si le connecteur est utiliser dans le cadre d'un traitement de dialyse, le dispositif de dialyse peut comprendre également des moyens de couplage de sorte que le capuchon (4) se souple sur le dispositif de dialyse afin de permettre le décapuchonnage dudit connecteur.

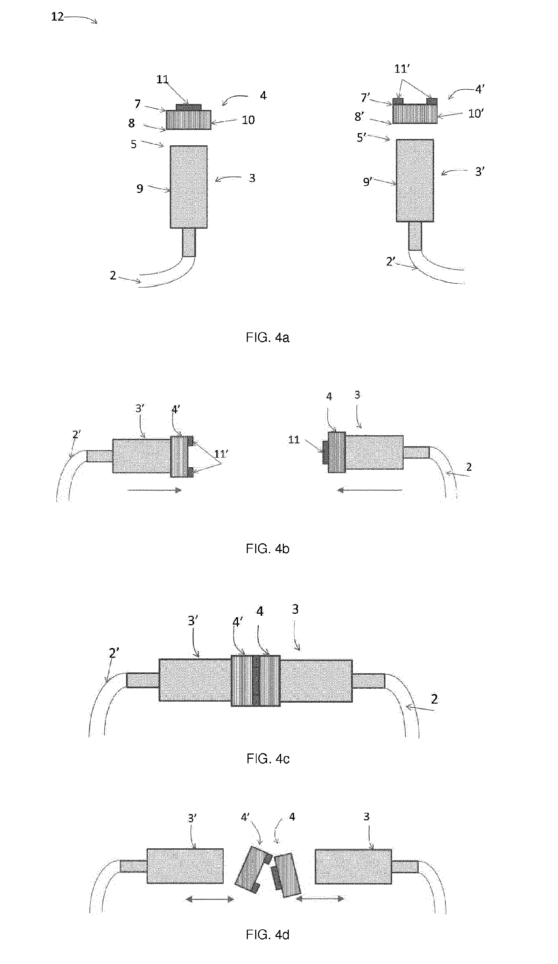

Dans un mode de réalisation exposé en figure 4a, un système (12) de connecteur comprend deux connecteurs distincts (3, 3') permettant de relier deux lignes fluidiques (2, 2'). Les extrémités distales (5, 5') desdits connecteurs (3, 3') sont conçues pour permettre la connexion desdits connecteurs (3, 3'). En outre, lesdites extrémités distales (5,5') peuvent être de formes identiques ou de formes différentes mais compatible, par exemple, l'un de type male et l'autre de type femelle. Les capuchons (4, 4') comprennent des moyens de couplage (11, 11') façonnés de manière à coupler les deux capuchons (4, 4') ensembles. Les figures 4b à 4d décrivent la manipulation pour le décapuchonnage des connecteurs. En figure 4b, le praticien peut maintenir un connecteur dans chaque main par le corps (9, 9') desdits connecteurs (3, 3'). Les deux connecteurs (3, 3') doivent se rapprocher afin que (en fig 4c) les deux capuchons (4, 4') se couplent ensemble.

Dans un mode de réalisation, un ensemble (1) peut comprendre en outre des moyens de libération (non exposé) qui s'activent dès que les capuchons entrent en contact et/ou se couplent de manière à libérer au moins un capuchon de son connecteur (3, 3').

Dans un mode de réalisation, le mécanisme de déverrouillage est activé par le praticien, par exemple grâce à un mouvement de rotation d'au moins un connecteur (3, 3') sur son axe principale.

Dans un mode de réalisation, après avoir déverrouillé les moyens de verrouillage d'au moins un ensemble, le praticien laisse tomber au moins un capuchon. La figure 4d schématise des capuchons (4, 4') qui ne restent pas couplés après le déverrouillage. Toutefois, dans un mode de réalisation, les moyens de couplage (11, 11') peuvent permettre de maintenir les capuchons solidaires au moins temporairement après le déverrouillage des capuchons (4, 4').

Lesdits moyens de couplages (11, 11') peuvent être des aimants et/ou être façonnés comme un crochet ou une autre forme permettant le couplage desdits capuchons entre eux.

Dans un mode de réalisation, les moyens de couplage (du ou des capuchons ou dudit dispositif cité en exemple plus haut) comportent un système de codage de manière à ce que seuls les codages compatibles permettent au capuchon de se coupler. Eventuellement le dit codage permet à certains capuchons de se coupler à une série d'autres capuchons, tandis que certains capuchons ne peuvent se coupler qu'à un nombre plus limités de capuchons.

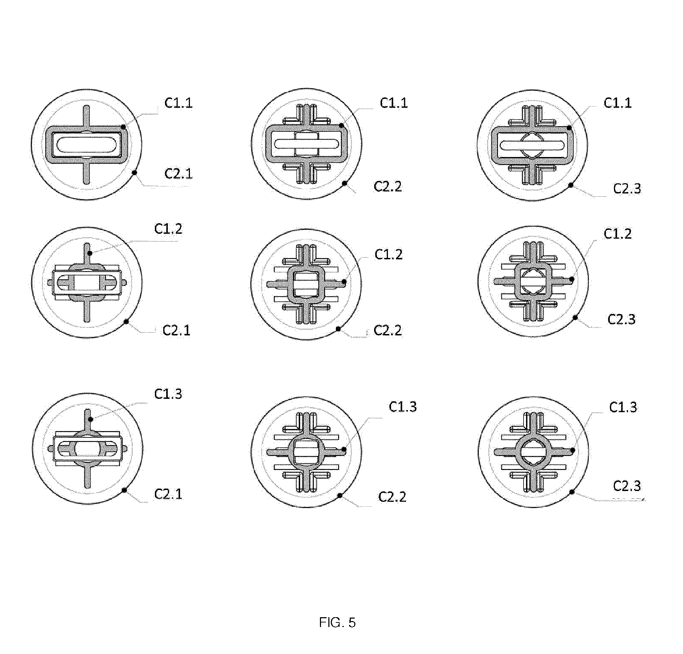

La figure 5 expose les différentes combinaisons possibles entre les différents moyens de couplage. Ainsi, les moyens de couplage C1.1 sont compatible avec les moyens de couplage du capuchon et/ou autre système/dispositif C2.1, C2.2 et C2.3. Cela signifie que le connecteur disposant d'un capuchon ayant des moyens de couplage de type C1.1 peut se connecter avec un système et/ou connecteur disposant d'un capuchon ayant des moyens de couplage de type C2.1, C2.2 ou C2.3. De même, C1.2 est compatible avec C2.2 et C2.3 mais incompatible avec C2.1. Et C1.3 est compatible avec C2.3 mais incompatible avec C2.1 et C2.2. Ce codage permet ainsi d'éviter toute erreur de connexion car les codages incompatibles ne permettent de décapuchonner les connecteurs. Par exemple, dans le cas où un connecteur est de type femelle et l'autre de type male, un capuchon de connecteur de type male ne pourrait se coupler avec un autre capuchon de connecteur de type male et/ou lorsque deux lignes fluidiques ne peuvent être compatible alors le système de codage empêcherait la connexion de ces deux lignes fluidiques ensemble car il serait impossible d'enlever normalement les capuchons. En plus du système de codage qui permet le couplage, le système de codage permet également de neutraliser l'élément de sécurité de sorte que même si en forçant le praticien arrive à les coupler, le système de codage empêche de lever la sécurité.

Dans un mode de réalisation, au moins un connecteur (3, 3') et/ou au moins un capuchon (4, 4') comprend des moyens d'entrainement de manière à éloigner au moins partiellement le capuchon du connecteur lorsque les moyens de verrouillage sont désactivés. Ces moyens d'entrainement peuvent permettre d'éjecter le capuchon de sorte que le praticien n'ait besoin de n'effectuer aucune autre manipulation pour libérer le connecteur ou simplement de déplacer le capuchon afin que le praticien n'ait qu'à orienter l'extrémité distale du connecteur vers le bas pour que le capuchon tombe.

Dans un mode de réalisation, les moyens de verrouillage sont détruits grâce à une manipulation du praticien de sorte que le capuchon se désolidarise du connecteur.

Dans un mode de réalisation, les moyens de verrouillage comprennent au moins un élément de sécurité de manière à empêcher la désactivation non intentionnelle des moyens de verrouillage. Cet élément de sécurité peut nécessiter une force minimum ou une manipulation spécifique pour être désactivé.

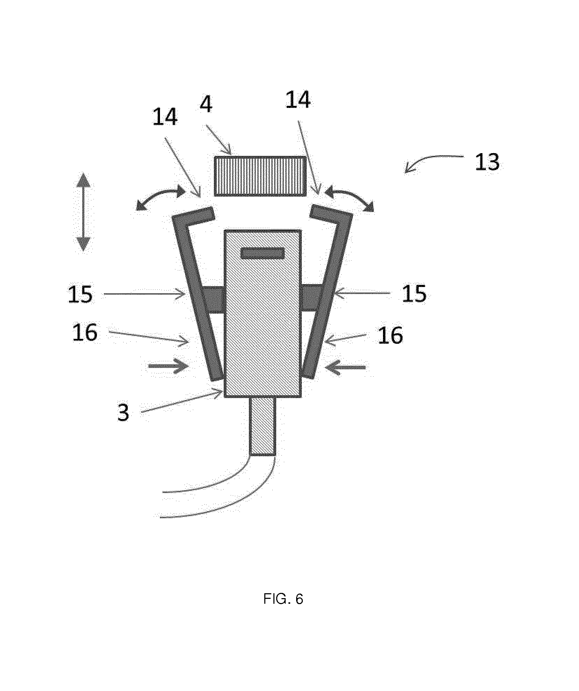

Dans un mode de réalisation schématisé en figure 6, les moyens de verrouillage (13) comprennent des moyens de retenus (14) agencés de manière à maintenir le capuchon (4) contre le connecteur (3) et des moyens de libération (15) activables grâce à un déclencheur (16). Un élément de sécurité peut être disposé de manière à retenir initialement le déclencheur (16). Sur cette figure conceptuelle, le pivot représente les moyens de libération (15), en appliquant une force contre le déclencheur (16) représenté par le bras des moyens de verrouillage (13), les moyens de retenus (14) en forme de crochet se déplacent pour libérer le capuchon (4). Dans ce mode de réalisation, l'ensemble connecteur capuchon peut également comprendre un ressort ou autre moyens d'entrainement comme une lame élastique qui permettrait d'éjecter ou déplacer le capuchon (4) lorsque les moyens de verrouillage (13) sont désactivés. Le capuchon (4) peut également comprendre de moyens de couplage. Ledit déclencheur (16) et/ou l'élément de sécurité peut être agencés sur et/ou dans le capuchon et/ou le connecteur.

Dans un mode de réalisation, les moyens de libération peuvent être activés en exerçant une force sur la face extérieure de l'extrémité distale du capuchon en direction du connecteur et les moyens d'entrainement peuvent être agencés de manière à éloigner ledit capuchon dudit connecteur au moins après activation des moyens de libération.

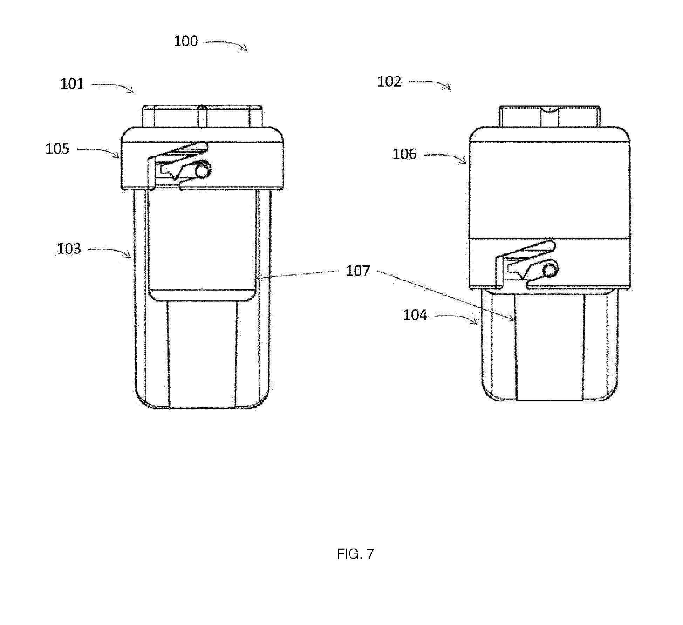

La description suivante s'applique à un mode de réalisation spécifique exposé par les figures 7 à 11:
La figure 7 présente le système de connecteur (100) qui comprend un premier ensemble (101) composé d'un connecteur de type femelle (103) également appelé premier connecteur et un capuchon (105) associé appelé également premier capuchon ainsi qu'un deuxième ensemble (102) composé d'un connecteur de type male (104) également appelé deuxième connecteur et un capuchon (106) associé appelé également deuxième capuchon.

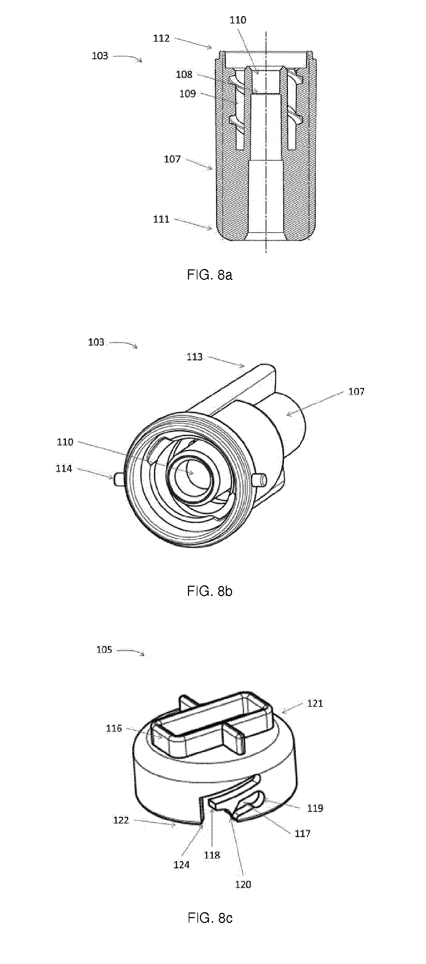

La figure 8a représente une coupe transversale du premier connecteur (103) composé d'un corps (107) traversé par un passage (110). L'extrémité proximale (111) du connecteur (103) est reliée de façon permanente à une ligne fluidique (non exposé). Le passage (110) peut être fermé grâce à l'aide d'une membrane (108) qui pourra être percée, déchirée et/ou ouverte par un autre connecteur, système ou dispositif.

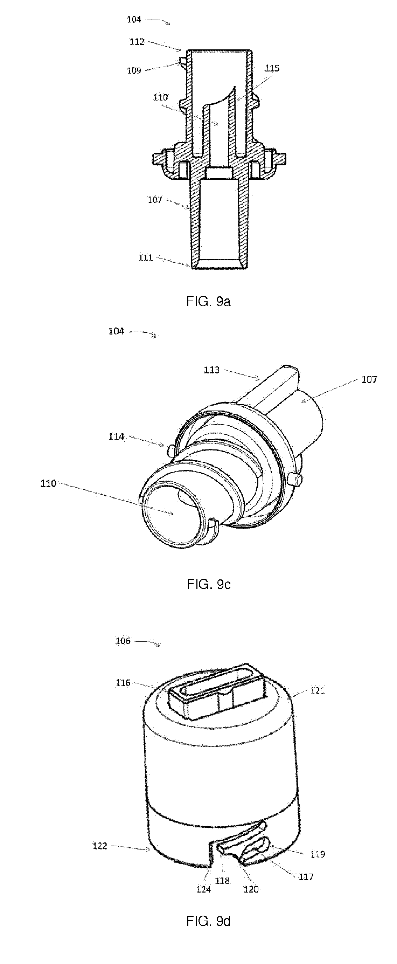

La figure 9a représente une coupe transversale du deuxième connecteur (104) composé d'un corps (107) traversé par un passage (110). L'extrémité proximale (111) du connecteur (104) est reliée de façon permanente à une ligne fluidique (non exposé). Ledit passage (110) du deuxième connecteur (104) peut comprendre un élément cylindrique (115) qui peut entrer dans le passage (110) du premier connecteur (103). Ledit élément cylindrique (115) peut comprend une extrémité pointu de manière à percer ou ouvrir la membrane (108) du premier connecteur (103).
Les figures 8b et 9b représentent une vue en perspective des connecteurs (103, 104)). Lesdits connecteurs (103, 104) peuvent comprendre au moins un élément faisant saillit sur leur corps (107) afin de former des ailettes (113) qui permettent de faciliter la manipulation desdits connecteurs (103, 104). Lesdits connecteurs (103, 104) peuvent en outre comprendre des ergots (114) façonné de manière à coopérer avec les moyens de verrouillage.
Les figures 8c et 9c exposent une vue en perspectives de capuchons (105, 106) desdits connecteurs (103, 104). Le capuchon (105, 106) comprend des moyens de couplage (116). Dans un mode de réalisation, ledit capuchon (105, 106) comprend les moyens de verrouillage qui peuvent être composé d'au moins une fente (117) s'étendant de l'extrémité proximale (122) du capuchon jusqu'à une butée (119). Ladite fente (117) peut être rectiligne et/ou incurvée. L'épaisseur d'au moins une fente (117) est au moins égale à la largeur de l'ergot (114) de sorte que l'ergot (114) puisse coulisser à l'intérieur de ladite fente (117). Ladite butée (119) peut être façonnée de manière à bloquer au moins temporairement ledit ergot (114). Ladite fente peut comprendre ou être composé d'une lame élastique (118) pouvant disposer d'un élément (120) faisant saillit de manière à bloquer ledit ergot (114) du connecteur (103, 104) entre l'élément (120) faisant saillit et ladite butée (119). Ladite fente (117) peut contenir une deuxième butée (124) opposée à la première butée (119) et disposée proche de l'extrémité proximale (122).

Dans un autre mode de réalisation, les ergots peuvent être agencés sur le capuchon et les moyens de verrouillage sur le connecteur.

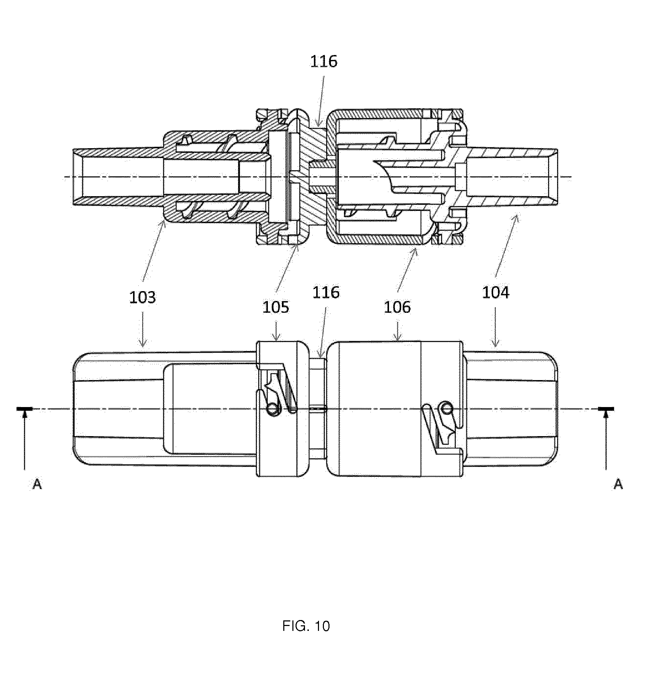

Dans un mode de réalisation, les moyens de verrouillage peuvent comprendre un système de filetage disposé sur les capuchons (105, 106) et/ou les connecteurs (103, 104); ledit système de filetage étant configuré de manière à se coupler et peut être composé d'au moins un ergot, d'un fliet et/ou d'une multitude de filet. Dans la figure 10, les deux capuchons (105, 106) sont montés et verrouillés sur leur connecteur (103, 104) respectif. Les deux capuchons sont couplés l'un à l'autre grâce aux moyens de couplage (116).

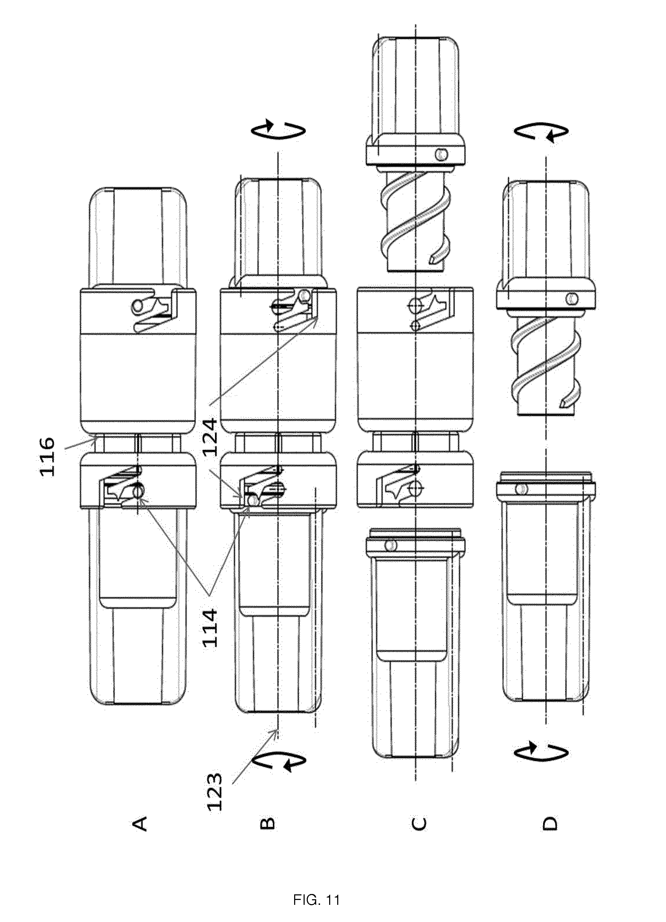

La figure 11 montre la manipulation nécessaire pour décapuchonner et connecter les connecteurs. En phase A, les deux connecteurs (103, 104) munis de leur capuchon (105, 106) respectif sont rapprochés l'un de l'autre afin de coupler les deux capuchons (105, 106) ensemble. Les ergots (114) sont durant cette phase contre la butée (119) de la fente (117). En phase B, le praticien fait pivoter selon l'axe (123) formé par le système au moins des deux connecteurs (104, 105). Les capuchons (105, 106) étant maintenus grâce au moyen de couplage, ne peuvent pivoter. Ainsi, les ergots (114) coulissent à travers la fente (117) jusqu'à l'élément (120) faisant saillit sur la lame élastique (118). Une force supplémentaire devra être appliquée afin de faire fléchir la lame élastique (118) pour qu'au moins un ergot (114) puisse être libéré de la fente (118). La deuxième butée (124) permet de retenir l'ergot (114) libéré afin que le processus de libération puisse s'effectuer sur l'ergot (114) de l'autre capuchon (105, 106). En phase C, les capuchons (105, 106) sont déverrouillés de leur connecteur (103, 104). Il suffit d'éloigner les deux connecteurs (103, 104) et laisser tomber les capuchons (105, 106). Dans un mode de réalisation, la lame élastique (118) agit également en tant que moyens d'entrainement afin de repousser le capuchon (105, 106) de son connecteur (103, 104) voir de l'éjecter. En phase D, les deux connecteurs (103, 104) sont rapprochés l'un à l'autre et connectés. Dans un mode de réalisation, les connecteurs (103, 104) comprennent un système de filetage façonné de manière à se coupler. Dans un mode de réalisation, ledit système de filetage qui permet de coupler les connecteurs est également utilisé pour coupler les connecteurs à leurs capuchons respectifs.

Dans un mode de réalisation, l'élément (120) faisant saillit sur la lame élastique (118) est façonné de manière à ce que l'ergot libéré (c'est à dire capuchon déverrouillé de son connecteur) ne puisse revenir dans sa position initiale contre la première buté (119). Dit autrement, une fois déverrouillé, l'élément (120) faisant saillit sur la lame élastique (118) empêche que le capuchon (105, 106) ne puisse être reverrouillé sur son connecteur (103, 104). Par exemple, la forme dudit élément (120) peut empêcher le chemin inverse ou la lame élastique (118) peut se rompre de sorte que le capuchon (105, 106) ne puisse plus tenir ou être reverrouillé sur le connecteur (103, 104).

Ce mode de réalisation est particulièrement pertinent pour éviter de toucher le capuchon. En effet, une manipulation simple et intuitive permet de décapuchonage des connecteurs. De plus, sans avoir à changer de les doigts de position sur le connecteur, il est possible de connecter de façon sure et propre les connecteurs.

## Revendications

1. Système de connecteur comprenant deux connecteurs conçus pour relier deux lignes fluidiques comportant respectivement un corps définissant un passage d'écoulement de fluide protégé par un capuchon disposé à l'extrémité libre du connecteur
dans lequel les connecteurs et/ou les capuchons comprennent des moyens de verrouillage conçus pour maintenir au moins temporairement et solidement les capuchons contre les connecteurs ;
**caractérisé par le fait que** les capuchons comprennent des moyens de couplage agencés de manière à coupler les capuchons desdits connecteurs entre eux au moins durant la désactivation des moyens de verrouillage.

2. Système selon la revendication 1, dans lequel au moins un des moyens de couplage comprend des moyens de retenus agencés de manière à maintenir au moins temporairement lesdits capuchons couplés de façon solidaire.

3. Système selon la revendication 1, dans lequel au moins un des connecteurs et/ou au moins un des capuchons comprend des moyens d'entrainement de manière à éloigner au moins partiellement le capuchon du connecteur lorsque les moyens de verrouillage sont désactivés.

4. Système selon la revendication 1, dans lequel les moyens de couplage comportent un système de codage de manière à ce que seuls les codages compatibles permettent aux capuchons de se coupler l'un à l'autre.

5. Système selon la revendication 1, dans lequel dans lequel les moyens de verrouillage comprennent un système de filetage disposé sur les capuchons et/ou les connecteurs ; ledit système de filetage étant configuré de manière à permettre un couplage.

6. Système selon l'une des précédentes revendications, dans lequel lesdits moyens de verrouillage comprennent au moins un ergot.

7. Système selon l'une des précédentes revendications, dans lequel lesdits moyens de verrouillage comprennent au moins une fente.

8. Système selon la revendication 1, dans lequel les moyens de verrouillage comprennent au moins un élément de sécurité empêchant toute désactivation non intentionnelle des moyens de verrouillage.

9. Système selon la revendication 8, dans lequel l'élément de sécurité comprend une lame élastique sur lequel un élément faisant saillit est façonné de manière à bloquer au moins temporairement lesdits moyens de verrouillage.

10. Système selon l'une des précédentes revendications, dans lequel l'élément de sécurité est désactivé en appliquant une force minimum.

11. Système selon la revendication 9, dans lequel l'élément de sécurité est façonné de manière à empêcher de reverrouiller le connecteur après avoir été déverrouillé.

12. Système selon la revendication 1, dans lequel les moyens de verrouillage comprennent :
● des moyens de retenus agencés de manière à maintenir le capuchon contre le connecteur,
● des moyens de libération activables grâce à un déclencheur et
● l'élément de sécurité () est disposé de manière à retenir initialement le déclencheur.

13. Système selon la revendication 12, dans lequel ledit déclencheur et/ou l'élément de sécurité sont agencés sur le capuchon et/ou le connecteur

14. Système selon la revendication 12, dans lequel les moyens de libération sont activés en exerçant une force sur la face extérieure de l'extrémité fermé du capuchon en direction du connecteur et les moyens d'entrainement sont agencé de manière à éloigner ledit capuchon dudit connecteur au moins après activation des moyens de libération.

15. Méthode de retrait de capuchons de connecteurs sans manipulation desdits capuchons, ladite méthode comprenant les étapes suivantes :
a. Maintenir les connecteurs par leur corps,
b. Mettre en contact les deux capuchons,
c. Déverrouiller les moyens de verrouillage,
d. Éloigner les deux connecteurs de manière à ce que les capuchons se désolidarisent des connecteurs.

16. Méthode selon la revendication 15, comprenant au moins une des étapes supplémentaires suivantes :
a. Coupler les capuchons à l'aide des moyens de couplage et/ou de codage
b. Désactiver l'élément de sécurité
